# EUROPEAN PATENT APPLICATION

(11) **EP 1 683 810 A1**
(43) Date of publication of application: **26.07.2006**
(21) Application number: 05001409.1
(22) Date of filing: 25.01.2005
(51) Int. Cl.: C07K 16/00, A01K 67/027

(54) **Method for producing antibodies**

(71) Applicant: Wengler, Georg S., 4894 Oberhofen (AT)
(72) Inventor: Wengler, Georg S., 4894 Oberhofen (AT)
(74) Representative: Schmitz, Hans-Werner

(57) **Abstract**

Immunosuppression, myeloablation, and the use of immunologically immature tissue can overcome major histocompatibility complex barriers by inducing tolerance. With the goal of inducing tolerance to BALB/c- derived murine hybridoma cells producing the 4C6 monoclonal antibody (mAb), we transplanted BALB/c fetal tissue into neonatal pigs during a regimen of low-dose conditioning with busulfan and cyclophosplramide. After the tolerance induction phase, animals received intraperitoneal injections of 4C6 mAb hybridoma cells. Evidence of persistence of injected cells over time was sought by molecular analysis peripheral blood for the presence of mouse genomic sequences and circulating 4C6 mAb. Persistence of donor polymerase chain reaction Signal during the entire duration of the study, detectable mAb titer for 6 weeks, and a twofold increase of mAb concentration after a boost hybridoma infusion was observed in one animal receiving six consecutive administrations of the conditioning regimen.

## Description

The present invention relates to a method for producing monoclonal antibodies according to claim 1, and to a bioreactor according to claim 7.

Obtaining stable chimerism through the induction of a state of immunologic tolerance is of major interest for the potential treatment of hematologic diseases and organ transplantation. In bone marrow transplantation, myeloablative conditioning followed by hematopoietic stem cell infusion results in engraftment of donor stem/progenitor cells followed by their differentiation into mature peripheral lineages. Conditioning, however, is associated with severe side effects.

The use of immunologically immature donor tissue transplanted into an immunologically immature recipient is sufficient to induce long lasting microchimerism in animals and humans (see WO 98/58963, the entire contents of which is incorporated herewith by reference). In animal models of in utero transplantation, it has also been demonstrated, that low-dose chemotherapy or TBI combined with peri- or postnatal reinfusion of donor hematopoietic cells can augment chimerism.

It is an object of the present invention to provide a method for producing monoclonal antibodies, which method provides relative simple test and production conditions of high efficiency and reproducibility.

This object is achieved by the features of claim 1.

Subclaims 2 to 6 relate to advantageous developments of said method.

Apart from the above-explained fundamentals, the present invention is based on the following concept and results:

In particular a xenogenic neonatal swine-mouse transplantation model aimed to tolerizing the recipient animal to monoclonal antibody (mAb)-producing murine hybridoma cells. The traditional method, growing murine hybridoma cells as ascites tumor in pristane-treated syngenic mice produces high yields of mAb. The extension of this approach to xenogeneic or allogeneic hybridoma usually results in rejection after intraperitoneal (IP) transplantation into murine recipients because of histocompatibility barriers. However, the engraftment of xenogenic hybridoma cells has been achieved in severe combined immunodeficiency mice with and without pretreatment. The transplantation procedure consisted of repetitions ("mini-blocks") of low- dose chemotherapy conditioning followed by a tolerogenic administration of BALB/c fetal tissue, that preceded the injection of BALB/c hybridoma cells.

Normal crossbred swines, Durok, Landrace and Large White strains were bred and maintained according to the veterinary regulations. The mini-block transplantation procedure started in neonatal pigs within 10 hours after birth and was repeated at two weeks intervals in one animal. Different concentrations of busulfan, (from 0 to 6 mg/kg body weight, BW) combined with cyclophosphamide (from 0 to 40 mg/kg BW) were tested in ten neonatal pigs. Two animals receiving 6 mg/kg busulfan in a single administration died of infections (data not shown). All other animals survived without any symptoms of drug side effects and were fertile, delivering healthy offspring. Each mini-block consisted of a single fixed dose of 2.4 mg busulfan administered on day 1. For dosage calculation (mg/kg BW) the weight of the animal was taken at the moment of injection. A single dose of 28 mg cyclophosphamide was administered on day 2. Cell suspensions were prepared from seven murine fetuses surgically isolated from time mated BALB/c female mice killed at 14 days post coitus and passed through a 120 µm nylon mesh. On day 3, such cells were transplanted IP into recipient animals. Transplantation of fetal cells in conditioned animals (no. 401 and 402) was repeated 6 times at 2 weeks intervals. Control animal 760 did not receive conditioning and was subjected to 3 injections of fetal cells. Control animal 208 did not receive conditioning nor injections of fetal cells.

At the end of the tolerance inducing protocol, animals 401, 402, 208 received two IP injections of 2-4x10⁸ hybridoma cells (4C6). The hybridoma 4C6, stabilized after hybridization of NSO myeloma cells with splenocytes of a BALB/c mouse immunized against bovine immunoglobulin (Ig)G and producing an anti-bovine IgG2 monoclonal antibody, was selected for transplantation experiments. Hybridoma cells were grown in vitro in Dulbecco's Modified Eagle's Medium (DMEM) supplemented with fetal calf serum 10%, hypoxanthine 1x10⁻⁹M, thymidine 1.6x10⁻⁵M, subcultures were performed every 2-3 days by dilution of cell suspension in fresh medium.

DNA was extracted from peripheral blood cells and amplified by polymerase chain reaction (PCR) using murine specific primers flanking an STR locus 1042 as previously reported. To increase sensitivity, Southern blot analysis was performed.

Furthermore, to demonstrate survival and activity of injected hybridoma cells, we titrated the presence of the monoclonal antibody 4C6 in pig sera by an indirect ELISA test, using microplates coated with purified bovine IgG, sequentially incubated with pig sera, goat IGs and antimouse IGs conjugated to peroxidase and substrate solution.

Animal 401 received six mini-blocks, pig 402, a sibling of 401, received one conditioning miniblock followed by 6 administrations of murine fetal tissue (Table 1). The animals treated with chemotherapy did not show any significant difference in body weight during the entire treatment (Table 1) nor clinical symptoms of common infections or graft-versus-host disease. However, animal 760, which received only fetal murine tissue without previous chemotherapy, presented with malnutrition and diarrhea and died after the third fetal tissue transplantation at the age of 42 days. Necroscopy revealed intraperitoneal fibrin clumps and locally enlarged lymph nodes, spleen, and suprarenal glands. The pathogenesis of these lesions remains unclear.

Two weeks after transplantation, animals received IP infusion of hybridoma cells. The hybridoma transplant procedure was repeated 5 weeks later.

The kinetics of the monoclonal antibody titer in the serum of transplanted animals during the 90-day period after hybridoma injections are shown in Picture 1. Minor fluctuations, just above the background level, were observed in animal 208, which was not treated with chemotherapy and fetal tissue, but received hybridoma cell transplantation. Low and transient levels of mAb after both hybridoma injections were detectable in animal 402. Animal 401 showed mAb serum levels comparable to pig 402 after the first hybridoma injection, however, the mAb concentration in pig 401 was four times higher than in the other animals after the second hybridoma injection. The mAb titers decreased to background levels during the 6 weeks following injection. At different time points, the engraftment of BALB/c in transplanted neonatal pigs was detected by PCR amplification (Picture 2A). The probe is murine specific (lane 1) as demonstrated by the absence of the PCR band in an untreated control pig (lane 2).

Animal 401 showed persistent chimerism after fetal murine tissue (lane 3), the first hybridoma cell injection (lane 5) and 17 days after the second hybridoma injection (lane 8). In contrast, chimerism in animal 402 was only temporary positive (lane 4 and 6) and disappeared after the second hybridoma injection (lane 9). In animal 208, which did not undergo the transplantation procedure, chimerism was demonstrated at a low transient level (lane 7). The time correlation between chimerism analysis, transplantation protocol and hybridoma infusion is summarized in Picture 2B.

To induce donor tolerance, cell suspension of entire donor fetuses were transplanted, providing an intensive exposure to a wide range of xenogenic fetal antigens into immature and immunosuppressed recipients. In the miniblock transplantation protocol, single, low-dose cyclophosphamide combined with busulfan in liquid form to avoid highly variable serum busulfan concentrations after oral administration were infused IP. Repeated, single, low-dose administrations allowed the increase of total dosage per Kg BW still avoiding severe side effects.

Animals received transplants with hybridoma cells of BALB/c origin and thus major histocompatibility complex identical with the fetal tissue. Consecutive transplantation of fetal BALB/c donor tissue lead to hematopoietic chimerism detected in the peripheral blood, indicating that donor cells actively migrated into the peripheral blood system. The signal of chimerism and the concentration of mAb after both hybridoma injections followed a similar pattern. Only chimeric animals showed mAb titers in their serum. Interestingly, higher total dosage per kilogram of BW of chemotherapy was observed to raise mAb titers. The disappearance of the mAb titers in the serum in both animals could be explained by immunologic donor rejection. The increase of the mAb titer after the second injection and the coexisting presence of chimerism in the animal receiving six miniblock transplants suggest the induction of tolerance.

The kinetic effect of mAb titer in the serum, in combination with PCR chimerism analysis constitute a read-out system for hybridoma-cell transplantation, giving information on the state of tolerance, engraftment and functionality.

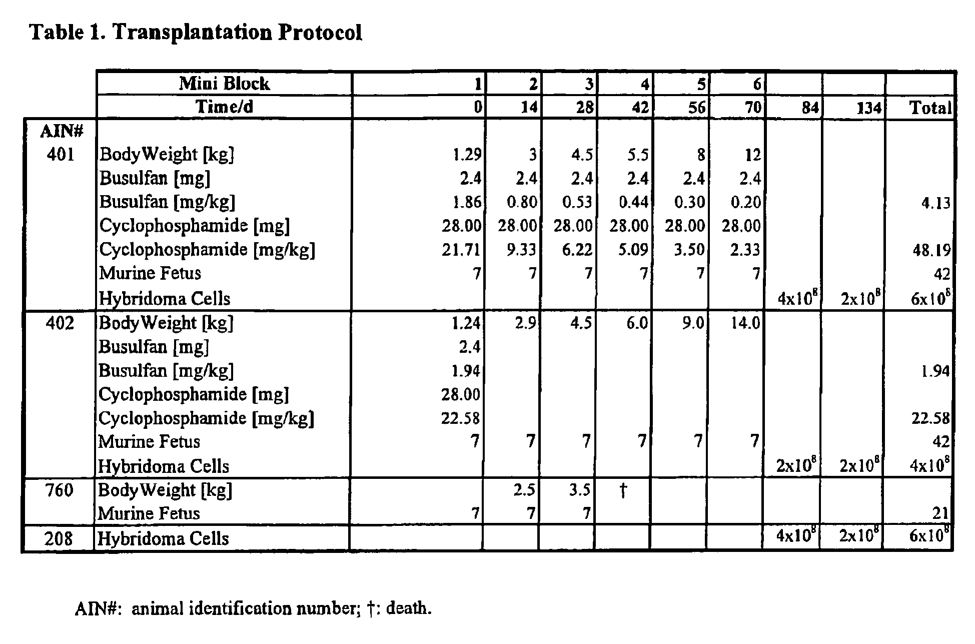

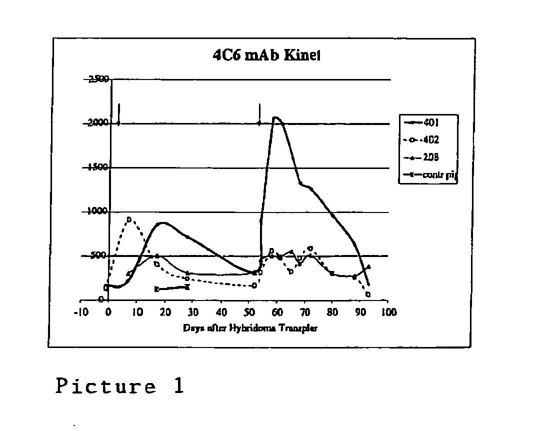

Picture 1. Kinetics of the monoclonal antibody (4C6 mAb) in serum of animals after hybridoma infusion, An indirect ELISA was used to titrate the presence of the monoclonal antibody 4C6 in hybridoma-injected pig sera. The concentration, expressed in OD recorded at serum dilution 1/10, of the monoclonal antibody (4C6 mAb), after the first hybridoma infusion, is comparable in animal 401 and 402 differing only in the persistence over time. In both animals the mAb concentration is higher than in the untransplanted animal 208 that received only hybridoma cells. After the second injection of hybridoma cells, only pig 401 showed an increase in the monoclonal antibody concentration. An untreated normal pig served as negative control. The arrows indicate the times of the hybridoma injections. 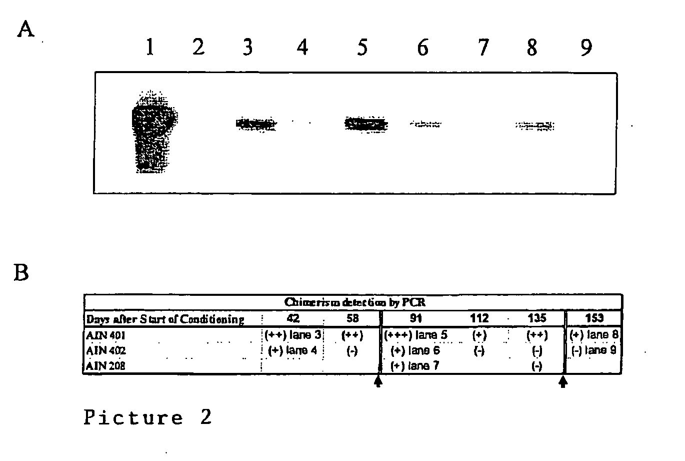
A
1 2 3 4 5 6 7 8 9

B
Chimerism detection by PCR
Days after Start of Conditioning | 42 | 58 | 91 | 112 | 135 | 153
AIN 401 | (++) lane 3 | (++) | (+++) lane 5 | (+) | (++) | (+) lane 8
AIN 402 | (+) lane 4 | (-) | (+) lane 6 | (-) | (-) | (-) lane 9
AIN 208 | | | (+) lane 7 | | (-) |

Picture 2
 Picture 2. Chimerism detection was performed by polymerase chain reaction (PCR) analysis at murine locus 1042. (A) Mouse DNA front peripheral blood mononuclear cells (PBMCs) (lane 1) and untreated pig's DNA from PBMCs (lane 2) represent the positive and negative control, respectively. Animals 401 (lane 3) and 402 (lane 4) demonstrated positive PCR signals after transplantation and after the first hybridoma injection (lane 5 and 6, respectively). The untreated animal, pig 208, shows a temporary PCR signal after the first hybridoma injection (lane 7). Pig 401 (lane 8) shows a positive PCR signal after the second hybridoma injection in contrast with pig 402 (lane 9). (B) Time correlation between transplantation protocol and hybridoma infusion and chimerism detection. The intensity of the PCR signal was arbitrarily classified as negative (-), weak (+), medium (++), and high (+++). Lane numbers correspond to the PCR signal shown in A. *(arrows)* Time points of two hybridoma injections, 84 and 135 days after start of the first miniblock, respectively.

Furthermore, the invention relates to a bioreactor according to claim 7 and the appended subclaims 8 to 10.

With the term "bioreactor" the present invention covers parts of animals which are kept alive with the aid of artificial life-sustaining equipment insofar as the body which is tolerant to a donor has a functioning blood circulation and a functioning Organ in which monoclonal antibodies can be produced after the injection of hybridoma cells which cause an antibody reaction.

This definition, however, covers a naturally living non-human mammal or a bird. In the case of mammals, particular preference is above all given to horses and sheep since particularly large amounts of antibodies can be produced in these animals.

Hence, with the definition "bioreactor", the present invention covers both of the above-explained possibilities, with the use of artificially life-sustained animal body parts having the advantage that the animal as such is dead, so that it no longer feels anything, in particular no pain. However, it should be noted that, in particular, suffering caused by pain can be kept very low even in cases where naturally living animals are used.

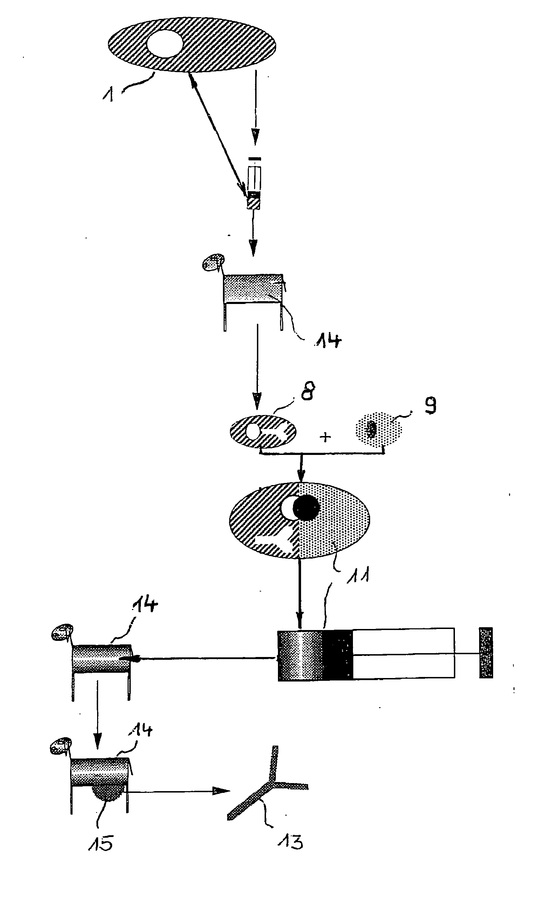

Further details, features and advantages of the present invention will become apparent from the following description taken in conjunction with the drawing, in which:

The only Figure shows a principle to explain a method of producing monoclonal antibodies according to the present invention.

The Figure diagrammatically shows a method of producing monoclonal antibodies 13. Mice of the Balb-C inbred strain serve, by way of example, as donors. The method is first characterized in that a tolerance must be induced in an animal organism. Here, a tolerance is induced with the aid of animal stem cells 1 of a donor, in particular the beforementioned Balb-C mouse.

In the next step of the method, B cells 8 taken from the donor are fused with tumor cells 9 (myeloma cells). This results in hybridoma cells 11 which are injected into the animal 14 having the tolerance (mouse tolerance). On account of its tolerance, this animal 14 does not reject the injected hybridoma cells 11, but rather forms an ascites 15 containing monoclonal antibodies, a blood serum containing said antibodies or also milk containing said antibodies when a cow is, for instance, used as the animal.

The monoclonal antibodies 13 can now be isolated in the known manner from the respective fluid, e.g. the ascites 15 shown in the Figure.

As for the explanations, in particular the method according to the described embodiment, the following should additionally be noted:

Immunosuppression, myeloablation, and the use of immunologically immature tissue can overcome major histocompatibility complex barriers by inducing tolerance. With the goal of inducing tolerance to BALB/c-derived murine hybridoma cells producing the 4C6 monoclonal antibody (mAb), we transplanted BALB/c fetal tissue into neonatal pigs during a regimen of low-dose conditioning with busulfan and cyclophosphamide. After the tolerance induction phase, animals received intraperitoneal injections of 4C6 mAb hybridoma cells. Evidence of persistence of injected cells over time was sought by molecular analysis peripheral blood for the presence of mouse genomic sequences and circulating 4C6 mAb. Persistence of donor polymerase chain reaction Signal during the entire duration of the study, detectable mAb titer for 6 weeks, and a twofold increase of mAb concentration after a boost hybridoma infusion was observed in one animal receiving six consecutive administrations of the conditioning regimen.

Since in the above explanation regarding the embodiment according to the Figure, mice of the inbred strain Balb-C were preferably used as animal donors, the cells of the original donor 10 cannot be distinguished from the cells of the other Balb-C mice, since all are "quasi" genetically and phenotypically (MHC) identical. This is the result of intensive inbreeding. During fusion of the B cells 8 with the tumor cells 9, which also derive from Balb-C mice, it is thus enough that these cells 9 derive from the so-called Balb-C mice.

As has been explained above, the above-mentioned hybridoma cells are cells which are produced by cell fusion. In the case explained with reference to the Figure (lymphocyte subtype) is preferably fused with a myeloma cell.

Furthermore, it should be taken into account for explanatory purposes that a tumor is a clump of cells having the following characteristics:
a) uncontrolled cell growth;
b) uncontrolled cell migration (metastases),
c) "immortality" (or better: "longevity");
d) partial disappearance of original cell characteristics in favor of new ones; and
e) all cells originate from a single degenerated cell (clonality).

A myeloma is a tumor of a B cell which is equipped with said characteristics (a-e).

Furthermore, to understand the present invention in an improved manner, it should be noted that a B cell produces a single antibody. Many B cells produce many different antibodies (= immunoglobulins are the fraction of proteins in plasma electrophoresis that contain all antibodies).

Since all tumor cells derive per definition from one cell, they only produce one antibody (M gradient in the plasma electrophoresis of such patients).

These characteristics are transferred to a normal donor B cell during fusion. Therefore, one talks about monoclonal antibodies (mAk). "Mono" means here "of one type" and "clonal" "derived from one cell".

The tumor or myeloma cell 9 which has been mentioned in connection with the explanation of the Figure may e.g. be derived from the myeloma cell line XG3AG8 of Balb-C mice, which is commercially available. Such myeloma cells can be used within the scope of the present invention as fusion partners.

For a supplementary disclosure and explanation, reference is made to the table shown on the next page and containing definitions of terms.

Furthermore, for a supplementary disclosure it should be added that after prior in vivo injection of the antigen into the donor aiming at specific donor B cell stimulation and subsequent in vitro fusion with the myeloma tumor cells and after growth carried out in the laboratory, by testing, those cells are determined that contain the specific antibodies stimulated by the antigen. The testing methods for determining the respective, specifically desired hybridoma cells are known per se.

| DEFINITION OF TERMS | | |
|---|---|---|
| Term | Abbreviation | Definition |
| Antigen | Ag | Molecules which react with antibodies. Their name results from their ability to produce antibodies. However, since some antigens cannot stimulate antibody production by themselves, only those antigens that can induce antibody production are designated as immunogens. |
| Antibody | Ak/Ab | Immune bodies; reaction products of the body cells to the stimuli of antigens against which they are specifically directed. Plasma proteins which specifically bind to molecules known as antigens. Antibody molecules are produced as a response to the immunization with an antigen. They are the specific molecules of the human immune reaction and bind and neutralize pathogens or prepare the source for absorption and destruction by phargocytes.The antibody molecule has a unique structure which enables the molecule to bind to specific antigens. All of the antibodies, however, have the same overall structure and are designated, an the whole, as immunoglobulins. |
| Monoclonal mAKImAB antibody | | Antibodies which are produced by a single nucleus of B lymphocytes. Monoclonal antibodies are normally produced through the production of hybrid antibody-forming cells from the fusion of myeloma cells with immune spleen cells. |
| B cells | | B cells or B lymphocytes constitute one of two main classes of lymphocytes. The antigen receptor an B cells, which is partly designated as a B cell receptor, is a cell-surface immunoglobulin molecule. Upon activation by an antigen B cells differentiate into cell-producing antibodies of the same specifity as that of the initializing receptor. |
| Balb-C | Balb-C | Specific inbred mouse strain. mouse |
| Hematopoiesis | | Formation of the cellular elements of blood, including the red blood cells, the leukocytes and the blood platelets. |
| Hybridoma | | Cell resulting from the fusion of a specific antibody-producing B cell with a myeloma cell. |
| Myeloma cell | | Tumor cell with B cell origin. |
| Stern cells | | Basophilic cells as the first stage of leukocyte development. |
| Stem cells, | | Cells capable of self-renewal and differentiation, |
| hematological | | used in the clinical field for hematopoietic reconstitution after irradiation or chemotherapy (bone marrow transplantation). |
| Tolerante | | State of specific immunological non reactivity after contact with a normally antigenic stimulus (in the case of a normal reaction to other antigens). |
| newborn (animal) | | date of birth until the age of about 6 months |

## Claims

1. A method for producing monoclonal antibodies, comprising the following steps:
a. transplanting stem cells (1) of a donor mammalian into a newborn animal organism (14) undergoing myeloablative and immunsupressive, preferably repeated low dose, chemotherapy;
b. fusing B cells (8) of the donor of step a. with tumor cells (9), preferably myeloma cells, resulting in hybridoma cells (11);
c. injecting the hybridoma cells (11) into the newborn tolerant animal organism (14); and
d. isolating monoclonal antibodies (13) from the resulting ascites (15), the resulting blood serum, the produced milk or the laid eggs of the tolerant animal (14).

2. The method according to claim 1, **characterized in that** sheep or cows or pigs or chicken are used as the recipient animal organisms (14).

3. The method according to claim 1 or 2, **characterized in that** a mouse, in particular of the inbred strain Balb-C, is used as the donor.

4. The method according to any one of claims 1 to 3, **characterized in that** the tolerant state is checked.

5. The method according to any one of claims 1 to 4,
**characterized in** stimulating the donor B-cells with an antigen prior to the fusing step b. of claim 1.

6. The method according to any one of claims 1 to 5,
**characterized in that** use is made of antigens (7) from pathogens of infectious diseases, in particular malaria, TBC, rabies, measles, tetanus, FSME, herpes and HIV or antigens against tumors or blood group antigens (rhesus factor D) or histocompatibility antigens (HLA antigens).

7. A bioreactor for producing monoclonal antibodies, comprising
at least one part of a life-sustained animal body having a functioning blood circulation; and
at least one functioning organ of the animal body in which, after injection of antibody-producing hybridoma cells of a donor, monoclonal antibodies can be produced or after injection of antigens antisera can be produced or are producible in the blood products,
wherein the animal body is tolerant to the donor of the cells.

8. The bioreactor according to claim 7, **characterized in that** the animal body is kept alive with technical equipment.

9. The bioreactor according to claim 7, **characterized in that** the part of the animal body is part of a non-human mammal or bird naturally kept alive.

10. The bioreactor according to any one of claims 7 to 9,
**characterized in that** the donor is a non-human mammal, e.g. a mouse, a ruminant, a horse or a goat, and that the animal body is tolerant to the donor.
